# EUROPEAN PATENT APPLICATION

(11) **EP 3 299 864 A1**
(43) Date of publication of application: **28.03.2018**
(21) Application number: 16782487.9
(22) Date of filing: 03.03.2016
(51) Int. Cl.: G02B 27/01, A61F 9/00

(54) **IMAGE ENHANCING EYEGLASSES STRUCTURE**

(30) Priority: 20.04.2015 CN 201510186261
(71) Applicant: Chen, Tai-kuo, New Taipei City, Taiwan (TW); Tsai, Hong-Bing, Yilan City, Yilan County Taiwan (TW)
(72) Inventor: Chen, Tai-kuo, New Taipei City, Taiwan (TW); Tsai, Hong-Bing, Yilan City, Yilan County Taiwan (TW)
(74) Representative: Wittmann, Günther
(86) International application number: PCT/CN2016/075441
(87) International publication number: WO 2016/169339

(57) **Abstract**

An eyeglasses structure enabling image enhancement comprises a frame body, two lens bodies jointed with the frame body, at least two transparent displays and at least one or else more than one image capture devices, wherein the interior of the frame body is connected to a processor used to perform image clarification processes on the image captured by the image capture device and forwardly extended from the frame body in order to improve its resolution, and also output the synchronously clarified image to the transparent display such that the image actually seen by the eyeball of a user through the lens body can overlap with the synchronously clarified image shown on the two transparent displays thereby sharpening the scenery image observed by the eyeball of the user through the lens body.

## Description

### BACKGROUND OF THE INVENTION

### 1. Field of the Invention

The present invention relates to an eyeglasses structure enabling image enhancement; in particular, it concerns an eyeglasses structure allowing the image actually seen by the eyeball of a user through the lens to overlap with the synchronously clarified image shown on the lens thereby clarifying the image observed by the eyeball of the user through the lens.

### 2. Description of Related Art

At present, various types of display devices have been developed because of technical advancements, ranging from compact hand-held displays to high quality display screens or even vivid stereo displays, whose lifelike image display quality allows people's unconstrained imagination to gallop freely.

Especially, so-called Head Mount Display (HMD) is widely considered as one of the major development topics for current display technologies. Typically, Google Glass is so far the most well-known item among various HMDs. Google Glass is a kind of wearable computer equipped with Optical Head Mount Display (OHMD) and designed to provide a computing device suitable for general consumer markets, wherein various information can be shown by Google Glass in a hand-free, smartphone-wise fashion. Therefore, users can communicate with Internet services via natural language voice commands.

With regards to the current Google Glass, the structure thereof is essentially characterized in that legs are configured on both sides of the frame and an electronic device having a battery is installed on a leg on one side of the frame, in which the electronic device extends to one side in front of the frame, a camera lens is installed in connection at an end thereof, a switch is set up in connection at the tail end of the leg in the electronic device, and also a screen is adjacently installed to the electronic device having a camera lens and extending to the front side of the frame.

However, in terms of Google Glass, for example, this type of HMD may encounter the following issues:
1. Google Glass may be quite inconvenient for users already wearing eyeglasses (i.e., users having myopic or hyperopic problems or other ocular issues requiring to wear eyeglasses); consequently, wearing the Google Glass directly on the head without the originally equipped eyeglasses may make the user unable to clearly recognize a view of a short or long distance;
2. It is possible to install the Google Glass onto a user's originally equipped eyeglasses; however, although the development for this type of HMD may demonstrate a light-weighted trend, it still has a certain volume and mass which may undesirably make the user feel uncomfortable if the Google Glass is to be placed onto the originally equipped eyeglasses;
3. Moreover, this sort of HMD does not include any auxiliary light sources in itself, so, during the daytime of insufficient luminance or nighttime, the camera lens on the HMD may be unable to provide clear images to the screen or even fail to take any pictures, thus that the user can not utilize this sort of HMD in some specific environments.

As a result, to address the aforementioned issues, it may be an optimal solution if it is applicable to currently existing technologies, in which a transparent display is jointly installed onto the lens of the common eyeglasses worn by general users, the processor in the frame of the eyeglasses can perform image clarification processes on the image captured and forwardly extended from the frame in order to improve its resolution, and output the synchronously clarified image to the transparent display such that the image actually seen by the eyeball of the user through the lens can overlap with the synchronously clarified image shown on the transparent display thereby clarifying the scenery image observed by the eyeball of the user through the lens.

### SUMMARY OF THE INVENTION

Accordingly, the present invention provides an eyeglasses structure enabling image enhancement which allows the image actually seen by the eyeball of a user through the lens to overlap with the synchronously clarified image shown on the lens so as to clarify the image observed by the eyeball of the user through the lens.

An eyeglasses structure enabling image enhancement capable of achieving the aforementioned objectives comprises: a frame body, the interior of which being connected to a processor including: a central processing module, used to control entire operations of the processor; an image processing module, connected to the central processing module, in which the image processing module is used to perform image clarification processes on the externally captured image information in order to improve its resolution; an image output module, connected to the central processing module and the image processing module in order to output the image-clarified externally captured image information as a synchronously clarified image; a remote connection module, connected to the central processing module thereby performing remote connections by means of wireless connection technologies; a power supply module, connected to the central processing module thereby connecting to an external device so as to store and supply electric power required for operations of the processor; two lens bodies, combined with the frame body and having a first surface and a second surface, wherein the distance between the second surface and the eyeball of a user is smaller than the distance between the first surface and the eyeball of the user; at least two transparent displays, respectively combined with the first surface, the second surface or the first and the second surfaces of the two lens bodies, and electrically connected to the image output module of the processor thereby displaying in real time the synchronously clarified image; at least one or else more than one image capture device, combined onto the frame body and electrically connected to the image processing module of the processor in order to capture the image forwardly extended from the frame body and convert the image into the externally captured image information for transferring to the image processing module; and the image actually seen by the eyeball of the user through the lens body can overlap with the synchronously clarified image displayed on the two transparent displays thereby clarifying the scenery image seen by the eyeball of the user through the lens body.

More specifically, the processor further comprises a capture angle adjustment module which is electrically connected to the central processing module and the image capture device in order to adjust the angle of the captured image such that the image seen at the perspective of the eyeball can be of the same angle as the perspective of the image captured by the image capture device and forwardly extended from the frame body thereby that the image actually seen by the eyeball of the user through the lens body can overlap with the synchronously clarified image displayed on the two transparent displays.

More specifically, the capture angle adjustment module can be pre-configured with a fixed eyeball perspective angle and perform pre-adjustments on the angle of the captured image based on the fixed eyeball perspective angle such that the image seen at the perspective of the eyeball can be of the same angle as the perspective of the image captured by the image capture device and forwardly extended from the frame body.

More specifically, the pre-configured eyeball perspective angle is the direct view angle.

More specifically, the eyeglasses structure enabling image enhancement is further capable of performing connections to the remote connection module of the processor by means of a software installed within an electronic device and transferring control commands for adjusting the angle of the captured image to the capture angle adjustment module by way of the central processing module in order to remotely adjust the angle of the captured image.

More specifically, the processor further comprises an output image adjustment module which is electrically connected to the central processing module and the image output module thereby adjusting the display status of the synchronously clarified image shown on the transparent display.

More specifically, the remote connection module of the processor can be connected to a cloud platform such that the cloud platform can transfer the digital display data to the processor and, through the output image adjustment module, the digital display data transferred by the cloud platform can be conjunctively shown over the synchronously clarified image on the transparent display.

More specifically, the digital display data of different angles can be respectively shown on different transparent displays, and the digital display data of different angles shown on different transparent displays can render image effects presenting a depth of field or stereo view, and also the digital display data of different angles may be the images processed by the cloud platform or otherwise the images captured by different image capture devices at different angles and then conjunctively processed by the processor.

More specifically, the processor can further transfer the externally captured image information to the cloud platform via the remote connection module.

More specifically, the display status shown on the synchronously clarified image of the transparent display that the output image adjustment module can adjust may include adjusting multiple display perspectives, adjusting display location, adjusting display size, adjusting wide angle, adjusting display contrast or adjusting display brightness.

More specifically, if any word or any replaceable object exists on the synchronously clarified image, then the output image adjustment module can replace the word in the synchronously clarified image shown on the transparent display with a clear word or replace the object with a built-in object.

More specifically, if the synchronously clarified image is a dimmed image, then the output image adjustment module can perform light compensations to the synchronously clarified image shown on the transparent display.

More specifically, the eyeglasses structure is further capable of performing connections to the remote connection module of the processor by means of a software installed within an electronic device and transferring control commands for adjusting the display status of the synchronously clarified image to the output image adjustment module by way of the central processing module in order to remotely adjust the display status of the output image.

More specifically, the lens body is a flat lens.

More specifically, the lens body is a curved lens.

More specifically, the frame body is further configured with at least one or else more than one sensor device electrically connected to the processor.

More specifically, any one of the sensor devices may be a sensor capable of detecting temperature, heartbeat, blood pressure, perspiration or providing a step counting function.

More specifically, the frame body is further configured with at least one or else more than one ear mountable device electrically connected to the processor, and the ear mountable device has a built-in battery thereby providing electric power to the power supply module.

More specifically, the frame body is further configured with at least one microphone device electrically connected to the processor, and the microphone device can transfer voice signals to the processor thereby voice controlling the operations of the processor.

More specifically, the frame body is further configured with at least one speaker device electrically connected to the processor.

More specifically, the at least two image capture devices can be further used to respectively capture images of different angles, and the images of different angles can be combined by the processor into a stereo image message or an image message presenting a depth of field.

More specifically, the output image adjustment module process the image in an array or matrix approach such that the image outputted to the at least two transparent displays have an image focusing effect.

More specifically, when at least one of the two lens bodies combine with more than one of the at least two transparent displays, images outputted to one or else two or more of the at least two transparent displays are processed in an array or matrix approach and have a multiple image focusing effect.

More specifically, the lens or the transparent displays can guide lights through collimation technologies.

More specifically, in order to guide lights, the collimation technology is a microlens array or a light well.

More specifically, the microlens array is further chamfered to adjust the direction of collimated lights.

More specifically, the two lens bodies or the at least two transparent displays are chamfered to adjust the direction of collimated lights to make two or more images overlapped.

More specifically, the at least two transparent displays are made with a collimation technology or a microlens array so that the at least two transparent displays have a light guiding effect.

### BRIEF DESCRIPTION OF THE DRAWINGS

Figure 1A shows a disassembled architecture diagram for the eyeglasses structure enabling image enhancement according to the present invention.
Figure 1B shows a assembled architecture diagram for the eyeglasses structure enabling image enhancement according to the present invention.
Figure 2 shows an architecture diagram for the processor inside the frame body of eyeglasses structure enabling image enhancement according to the present invention.

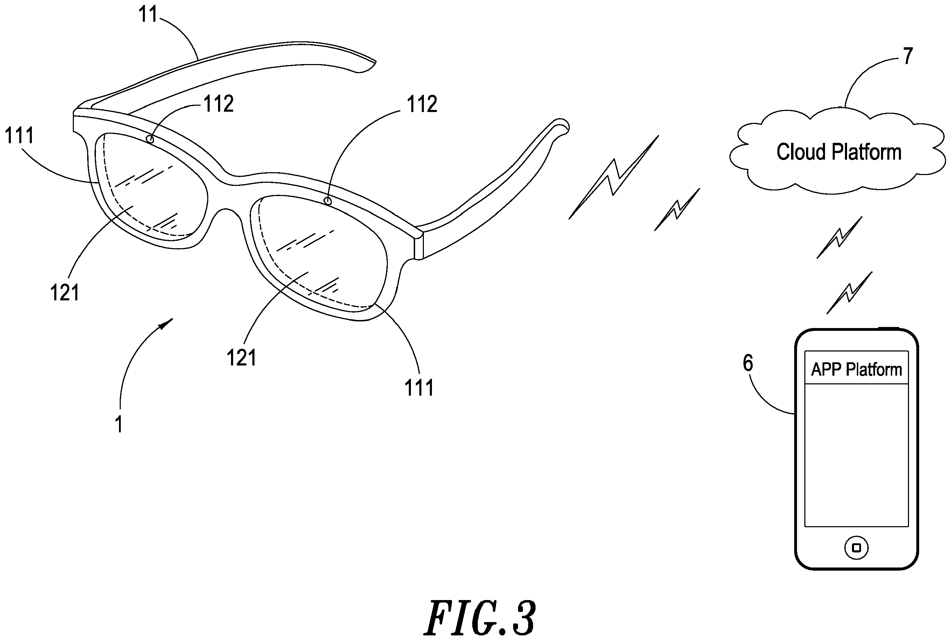

Figure 3 shows a remote control architecture diagram for the eyeglasses structure enabling image enhancement according to the present invention.
Figure 4A shows a conventional myopic eyeball focusing diagram.
Figure 4B shows a diagram for a first embodiment of the eyeglasses structure enabling image enhancement according to the present invention.
Figure 5A shows a conventional hyperopic eyeball focusing diagram.
Figure 5B shows a diagram for a second embodiment of the eyeglasses structure enabling image enhancement according to the present invention.
Figure 6A shows a diagram for a conventional myopic concave lens focusing correction.
Figure 6B shows a diagram for a third embodiment of the eyeglasses structure enabling image enhancement according to the present invention.
Figure 7 shows a diagram for another embodiment of the eyeglasses structure enabling image enhancement according to the present invention.
Figure 8 shows a diagram for another embodiment of the eyeglasses structure enabling image enhancement according to the present invention.

### DETAILED DESCRIPTION OF THE PREFERRED EMBODIMENTS

Other technical contents, aspects and effects in relation to the present invention can be clearly appreciated through the detailed descriptions concerning the preferred embodiments of the present invention in conjunction with the appended drawings.

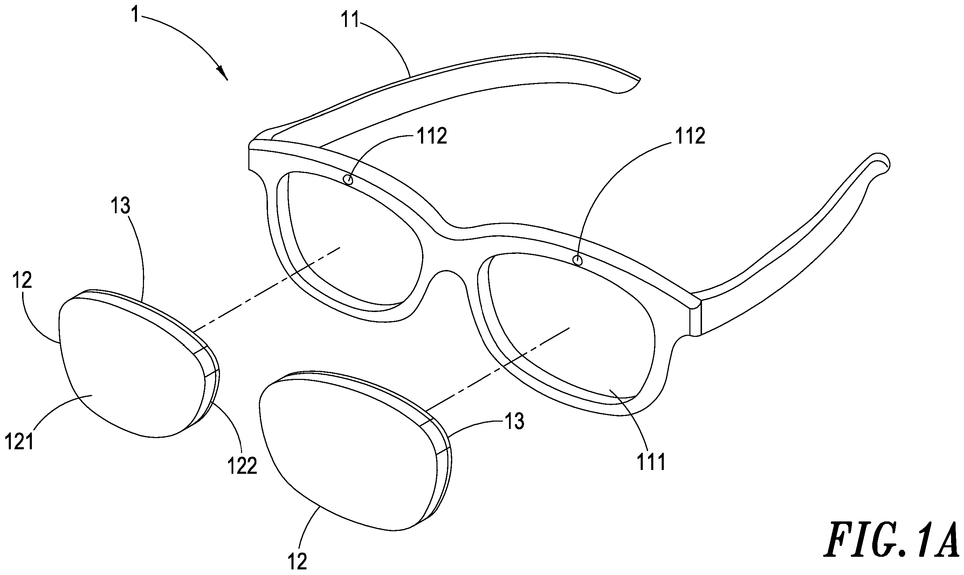

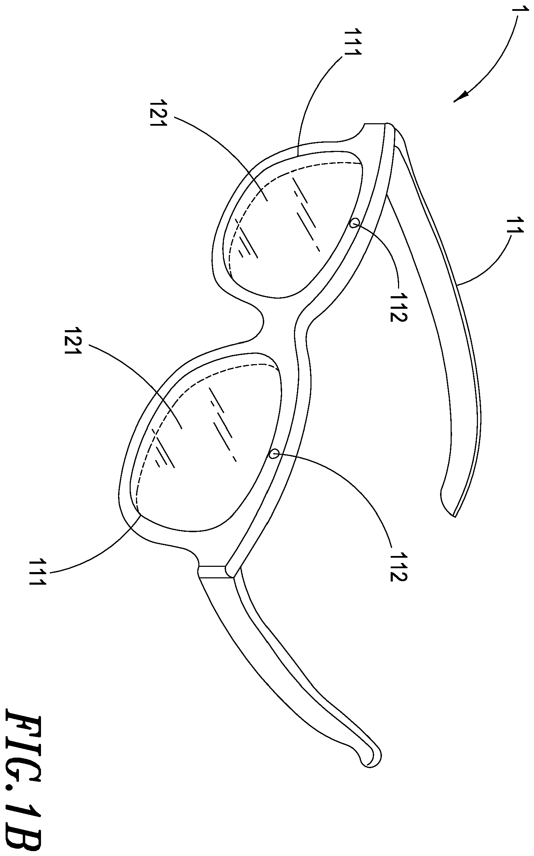

Refer now to Figures 1A and 1B, wherein a disassembled architecture diagram as well as a assembled architecture diagram for the eyeglasses structure enabling image enhancement according to the present invention are respectively shown. From the Figures, it can be appreciated that the eyeglasses structure enabling image enhancement 1 comprises a frame body 11, two lens bodies 12 combined to the frame hole 111 of the frame body 11, at least two transparent displays 13 and two image capture devices 112, wherein the frame body 12 has a first surface 121 and a second surface 122, the distance between the second surface 122 and the eyeball of a user is smaller than the distance between the first surface 121 and the eyeball of the user, and the transparent display 13 can be jointed onto the second surface 122 of the lens body 12 by means of the attachment, plating or coating application method (or otherwise it can be combined onto the first surface 121, or the transparent displays 13 are combined onto both the first surface 121 and the second surface 122, and the transparent display 13 features a display technology capable of active illumination display, rather than the image projection technology); besides, the lens body 12 may be a flat lens or a curved lens (and the curved lens may be a concave lens, a convex lens, a concave-convex lens or a lens having other curved surfaces.)

The image capture device 112 is used to capture the image forwardly extended from the frame body 11 and convert the image into the external captured image information to transfer to the image processing module 1132, and such two image capture devices 112 can be installed individually and correspondingly right above the two eyeballs of the user, but possibly on the periphery around the frame hole 111 of the frame body 11 as well.

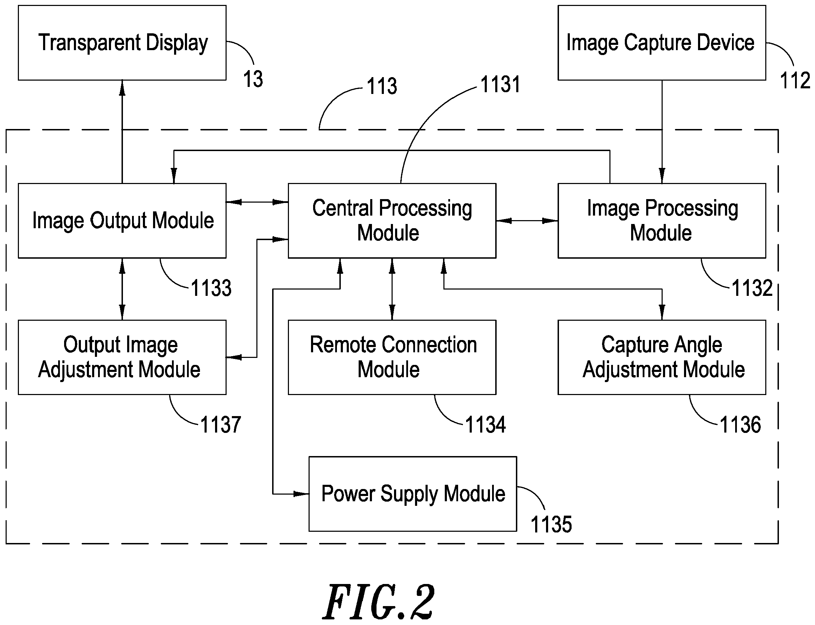

Meanwhile, the frame body 11 internally has a processor 113 and includes a hollow glass frame such that the inside of the frame body 11 can be configured with electric circuits and wires. From Figure 2, it can be seen that the processor 113 comprises a central processing module 1131, an image processing module 1132, an image output module 1133, a remote connection module 1134, a power supply module 1135, a capture angle adjustment module 1136 and an output image adjustment module 1137, wherein the central processing module 1131 can be used to control the operations of the entire processor, and image clarification processes can be performed on the externally captured image information from the image capture device 112 by means of the image processing module 1132 so as to improve its resolution.

Herein, the remote connection module 1134 allows remote connections by means of wireless connection technologies. Besides, the power supply module 1135 can be connected to an external device in order to store and provide electric power required for operations of the processor, and a power supply socket (not shown) electrically connected to the power supply module 1135 can be additionally installed on the frame body 11 thereby externally connecting a power line or USB transmission line for power recharging. Moreover, the power supply module 1135 (battery) can be devised as a detachable component placed on the frame body 11 such that, after taking down the detachable component, it is possible to replace the power supply module 1135 (battery).

Furthermore, the image output module 1133 outputs the image-clarified externally captured image information as a synchronously clarified image to the transparent display 13, and the user wearing the eyeglasses structure enabling image enhancement sees the synchronously clarified image on the transparent display 13 (but it is also possible to directly connect to the remote connection module 1134 in the eyeglasses structure enabling image enhancement 1 directly by way of an APP platform running in the hand-held device 6), and as the cloud platform 7 connected to the remote connection module 1134 in the eyeglasses structure enabling image enhancement 1, the user can operate the APP platform to input adjustment commands for controlling the output image such that, upon adjusting, the adjustment commands can be delivered to the output image adjustment module 1137 through the cloud platform 7, the remote connection module 1134 and the central processing module 1131 so as to adjust the display status of the synchronously clarified image in accordance with the control commands, so the user can continue to control the APP platform for fine tuning operations while the user is watching the post-adjustment status, until the user determines it is satisfactory.

The display status set forth herein may be multiple display perspectives (i.e., in addition to the eyeball straight view perspective, images at plural perspectives around the eyeball straight view perspective are also provided, and the user is allowed to perform fine tunings on the collimation of the image observed at different eyeball perspectives by means of UP, DOWN, LEFT, UPPER LEFT, LOWER LEFT, RIGHT, UPPER RIGHT, LOWER RIGHT perspectives of the user's own eyeball), adjusting display location (fine tunings in UP, DOWN, LEFT, UPPER LEFT, LOWER LEFT, RIGHT, UPPER RIGHT, LOWER RIGHT etc., at least 8 directions), adjusting display size (zoom in or zoom out), adjusting display contrast, adjusting display brightness (brighter or dimmer) or adjusting wide angle. Moreover, if the synchronously clarified image has any word, the adjustment control commands may include commands for word replacements such that the output image adjustment module 1137 can replace the word in the synchronously clarified image on the transparent display 13 with a clear word. Besides, in case the observed scenery is in a daytime of insufficient luminance or nighttime, the synchronously clarified image may become dimmer; hence, the user can also use the APP platform to input commands such as light compensation such that the output image adjustment module 1137 performs a light compensation operation to the synchronously clarified image on the transparent display thus also achieving the night vision function.

In addition to the aforementioned word replacement, if any replaceable object is found on the synchronously clarified image, it can be also replaced by a built-in object of the processor 113, in which such built-in objects may include, for example, graphics, icons, face images, words, buildings, biological characteristics etc.

Meanwhile, although the above-said image processing module 1132 and output image adjustment module 1137 are configured within the frame body 11, the remote connection module 1134 may also upload the captured image to the cloud platform 7, and since the cloud platform 7 can achieve the functions of the image processing module 1132 and output image adjustment module 1137, the cloud platform 7 can substitute the image processing module 1132, the capture angle adjustment module 1136 and output image adjustment module 1137, and then return the processed image back to the remote connection module 1134 of the frame body 11 such that the processed image can be directly outputted to the transparent display 13.

In addition, the output image adjustment module 1137 can also process the image in an array or matrix approach such that the image outputted to the transparent display 13 can present an image focusing effect when the user's eyeball is watching. Also, if multiple layers of transparent displays 13 are placed on the lens body 12, since the image outputted to one or else two or more of such multiple layers of transparent displays 13 is processes in the array or matrix fashion, the multiple image focusing effect can be achieved.

Moreover, it is also possible to apply various collimation technologies, such as a microlens array or a light well technology, on the lens body 12 or the transparent display 13 to guide lights. The microlens array changes lights through at least one lens, and the light well technology makes the lights go straight through a light well.

The microlens can be further chamfered to adjust the direction of collimated lights. Besides that, during the manufacturing process of the transparent display 13, it is also possible to apply the collimation technologies or the microlens array such that the off-factory transparent display 13 has a structure similar to a microlens array or a light well and has a light guiding effect.

In addition, the lens body 12 or the transparent display 13 can be chamfered, and the chamfers of the lens body 12 or the transparent display 13 can adjust the direction of collimated lights to make two or more images overlapped.

Furthermore, in case the images respectively shown on the two different transparent displays 13 are captured at different angles, when a user watches such two different left and right transparent displays 13 respectively with the left eye and the right eye, it allows the user to experience an image effect of depth of field or stereo sense, in which these images of different angles may be individually obtained by more than two image capture devices 112 (and the image capture devices 112 can be set to capture the image at a specific angle.)

Additionally, it is possible to apply the images respectively captured by two or more image capture devices 112 at different angles, and the processor 113 then merges these images captured at different angles to acquire an image message containing the feature of depth of field or stereo sense (i.e., combined into one image including two or more angles) and outputted to the transparent display 13 (the image including two or more angles can be individually shown on different transparent displays 13); the aforementioned combination process can also be completed at the cloud platform 7 and then the processed image is transferred to the eyeglasses structure enabling image enhancement 1.

In addition, it is also possible on the cloud platform 7 to capture or download the 2D image (digital display data) stored in the cloud platform 7 via the remote connection module 1134 of the frame body 11, and then process the 2D image by means of the output image adjustment module 1137 into images of different angles such that different transparent displays 13 can respectively show an image of different angle (digital display data) in order to render image effects presenting a depth of field or stereo view. Moreover, the cloud platform 7 can also save the processed digital display data of different angles or otherwise directly upload the 2D image captured by the image capture device 112 to the cloud platform 7 such that the cloud platform 7 can process the 2D image into images of different angles and then send them back to the remote connection module 1134 of the frame body 11 so as to directly output the images of different angles to different transparent displays 13.

Moreover, since the quality of the image capture device 112 may influence the resolution in the captured image and the quality of the transparent display 13 may affect display resolution in the synchronously clarified image, suppose it is intended to increase the resolution of the image, it is also possible to upgrade the image capture device 112 and the transparent display 13 so as to enhance the resolution of output images with better hardware.

The capture angle of the image capture device 112 may not be identical to the perspective of the user's eyeball; however, if the capture angle of the image capture device 112 can be the same as the perspective of the user's eyeball, the image actually seen by the eyeball of the user through the lens body 12 can overlap with the synchronously clarified image shown on the two transparent displays 13. Therefore, in generally, the capture angle adjustment module 1136 can pre-configure a fixed eyeball perspective angle (e.g., a straight view angle) and perform pre-adjustments on the angle of the captured image in the image capture device 112 based on the fixed eyeball perspective angle such that the image seen at the perspective of the eyeball can be of the same angle as the perspective of the image captured by the image capture device and forwardly extended from the frame body 11.

The aforementioned situation can be the factory presets of the product, so when the user actually applies the eyeglasses structure enabling image enhancement 1, in case it is found that the image shown on the transparent display 13 may not overlap with the scenery image actually seen by the eyeball, this indicates there exists an error in the angle of captured image from the image capture device 112. As such, the user can connect to a cloud platform 7 by way of the APP platform in the hand-held device 6 (but also possibly connect directly to the remote connection module 1134 in the eyeglasses structure enabling image enhancement 1 via the APP platform in the hand-held device 6), and the cloud platform 7 may connect to the remote connection module 1134 in the eyeglasses structure enabling image enhancement 1 such that the user can operates the APP platform to input control commands to the capture angle adjustment module 1136 thereby indirectly adjusting the angle for the images to be captured by the image capture device 112. Hence, upon adjusting via the APP platform, the image capture device 112 can turn the lens, the image shown on the transparent display 13 moves as well, until the user feels the image actually seen by the eyeball through the lens body overlaps with the synchronously clarified image shown on the two transparent displays thereby completing the correction action (such a condition indicates that the image seen at the perspective of the eyeball is of the same angle as the perspective of the image captured by the image capture device 112 and forwardly extended from the frame body.)

Additionally, the image capture device 112 can be further configured with a function for capturing images of wavelengths other than visible light such that the image capture device 112 can capture images on wavelengths out of the visible light spectrum so images can be clearly captured during nighttime (e.g., night vision function), or acquire ultraviolet light images and so forth; since the present invention is also applicable to ultraviolet light images, it is possible to further design ultraviolet light alarm software to conjunctively operate on the captured images.

Besides, the image capture device 112 further provides a zoom in / zoom out function, just like a camera, thereby enlarging an image to be captured at a remote position (similar to a telescope) or directly magnifying an image at a closer distance (similar to a magnifier) such that clear images can be successfully captured no matter at a farther or a closer distance.

The output image adjustment module 1137 can be also configured with an eyeball tracing function in order to trace the perspective of the eyeball anytime thereby adjusting the angle of the image to be captured by the image capture device 112 based on the perspective of the eyeball, so the adjustments need not to be manually performed by a user via the APP platform remotely, but can be automatically done.

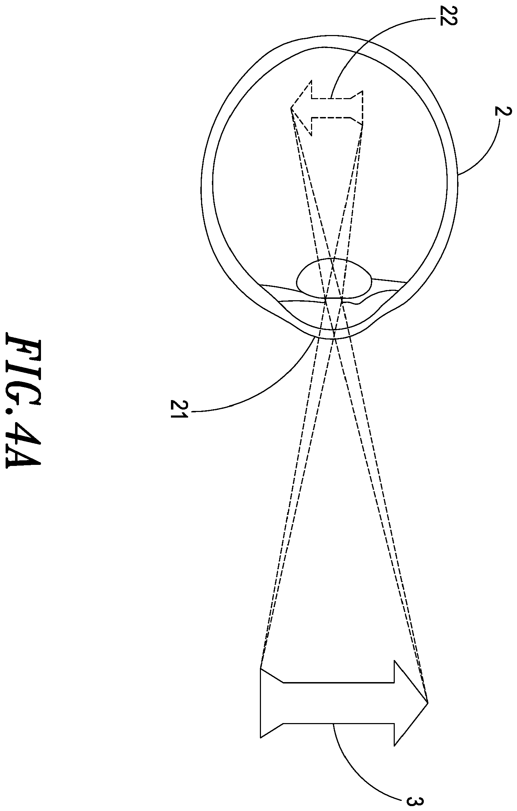

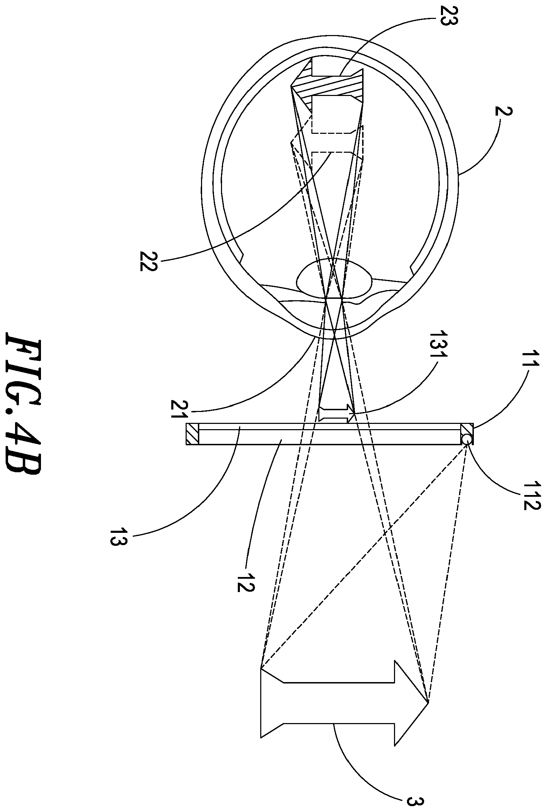

Next, Figure 4B shows a first embodiment of the present invention, while Figure 4A shows a general eyeball myopia diagram. Because the eyeball 2 is exceedingly long (i.e., the distance from the ocular lens to the retina is too long), or otherwise the zooming function of the ocular lens for remote objects weakens so that its far point may become closer, an obscure scenery image 22 generated through the cornea 21 by a scenery object 3 exceeding the far point may fall before the retina such that a vague image is created on the retina thus leading to unclear vision. From Figure 4B, however, it can be appreciated that, with the eyeglasses structure enabling image enhancement 1 where a transparent display 13 is installed in front of the eyeball 2, although the scenery object 3 seen by the eyeball 2 through the lens body 12 (flat lens) is also an obscure image on the retina, since the image capture device 112 can directly capture the image of the scenery object 3 and then perform image clarification processes thereon in order to improve its resolution, it is possible to render the synchronously clarified image 131 on the transparent display 13.

In addition, since the synchronously clarified image 131 is rendered very close to the eyeball 2 and the synchronously clarified image 131 can form a clear scenery image 23 on the retina of the eyeball 2 such that the processed image can overlap on the retina, in which, although an obscure scenery image 22 does exist before the clear scenery image 23, the mechanism in the eyeball 2 drives it to capture the clear image such that the focus of the eyeball 2 is directed to the clear scenery image 23 and ignores the obscure scenery image 22, thus that the final image being watched is the clear scenery image 23 (i.e., the obscure scenery image 22 can be considered as being overlapped and replaced.) In this way, the present invention allows myopic users to experience vision correction effects without wearing myopic eyeglasses (that is, for myopic users, far vision is vague but near vision is clear, the image capture device 112 captures the far scenery image which is then rendered in the eyeball 2 of the user through the transparent display 13, so the eyeglasses structure enabling image enhancement 1 can make the far scenery image become very clear.)

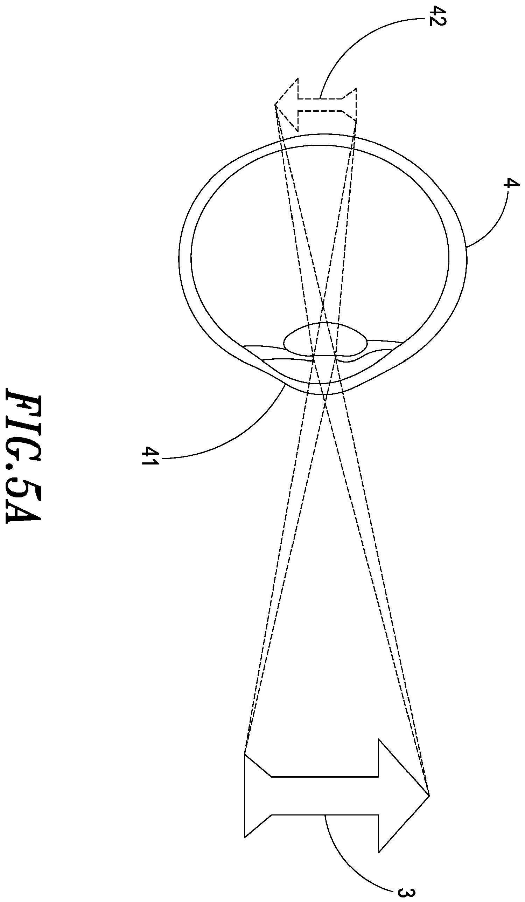

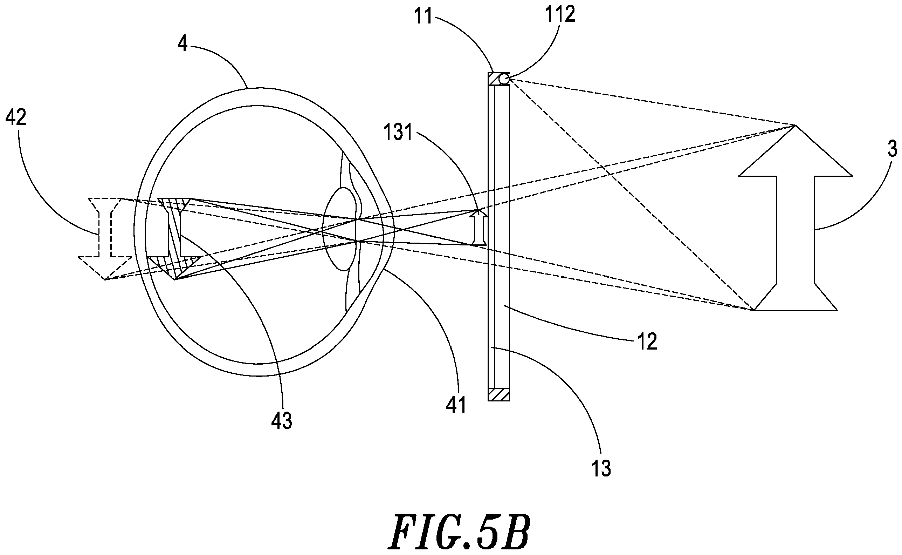

Next, a second embodiment of the present invention is shown in Figure 5B, and Figure 5A illustrates a conventional hyperopic eyeball focusing diagram, wherein because the eyeball length of the eyeball 4 is too short, or else the zooming function of the ocular lens for near objects weakens so that its clear vision distance becomes farther, the obscure scenery image 42 of the scenery object 3 generated through the cornea 41 falls behind the retina thus leading to unclear eyesight. From Figure 5B, however, it can be appreciated that, with the eyeglasses structure enabling image enhancement 1 where a transparent display 13 is installed in front of the eyeball 2, although the scenery object 3 seen by the eyeball 4 through the lens body 12 (flat lens) is also an obscure image on the retina, since the image capture device 112 can directly capture the image of the scenery object 3 and then perform image clarification processes thereon in order to improve its resolution, it is possible to render the synchronously clarified image 131 on the transparent display 13.

Hyperopic users may have an obscure near-distance vision so they tend to place an object farther to see it clearly; as such, when a clear image is formed in front of the eyeball 4, the synchronously clarified image 131 can create a clear scenery image 43 on the retina of the eyeball 4 such that the processed image can overlap on the retina, in which, and although an obscure scenery image 42 does exist behind the clear scenery image 43, the mechanism of the eyeball 4 causes it to capture the clear image thus that the obscure scenery image 42 is ignored but the eyeball concentrates on the clear scenery image 43 thereby allowing a hyperopic user to experience the vision correction effect without wearing hyperopic eyeglasses.

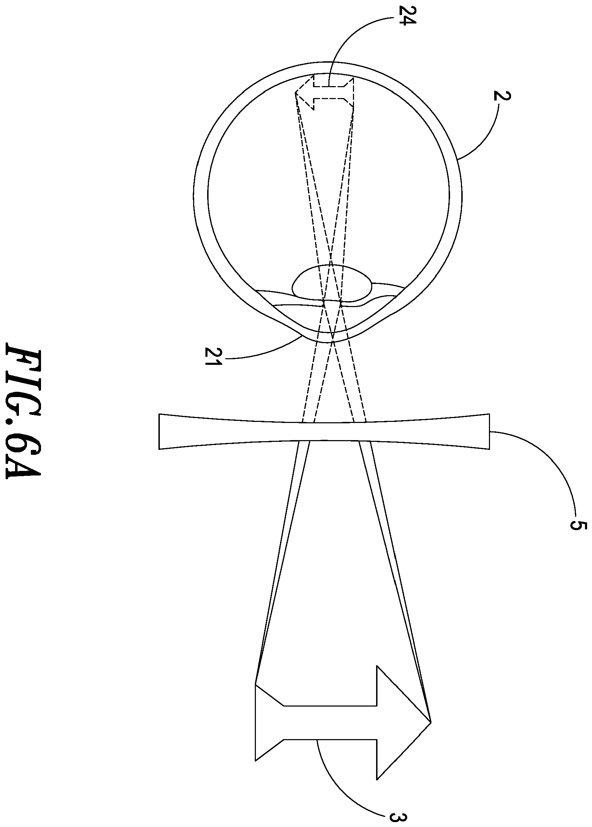

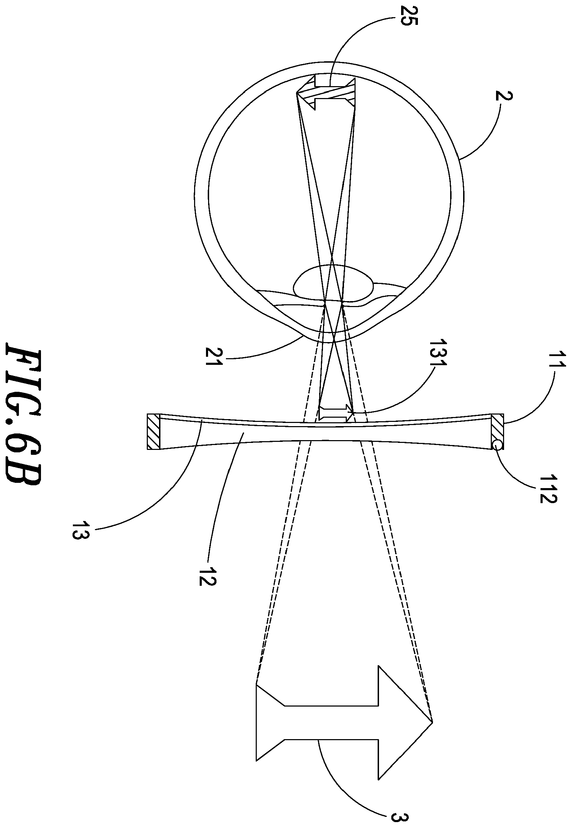

Following this, Figure 6B shows a third embodiment of the present invention, in which Figure 6A shows a diagram for general myopia with concave lens vision correction. It can be understood from the Figure that, when a user wears a pair of eyeglasses including a concave lens 5, the eyeball 2 can see clearer scenery images 24 as far as possible; however, human eyeballs have their limits, and the observed view may become more obscure as the distance extends farther. Nonetheless, from Figure 6B, it can be appreciated that, by wearing the eyeglasses structure enabling image enhancement 1, a transparent display 13 is placed before the eyeball 2; hence, although the scenery object 3 may be very far away, if the image capture device 112 can capture an image for remote scenery and ameliorate its resolution by means of image clarification processes, the synchronously clarified image 131 shown on the transparent display 13 is equivalent to taking the remote scenery image directly to the front side of the eyeball 2 for displaying such that the processed image can overlap on the retina, so the scenery image 25 can be clearly rendered on the retina of the eyeball 2 even if the scenery image is beyond the visible range of the eyeball.

In addition to the concave lens vision correction, for other ocular issues, no matter with or without curved lens vision corrections, it can be jointed with the curved lens to achieve the same effect.

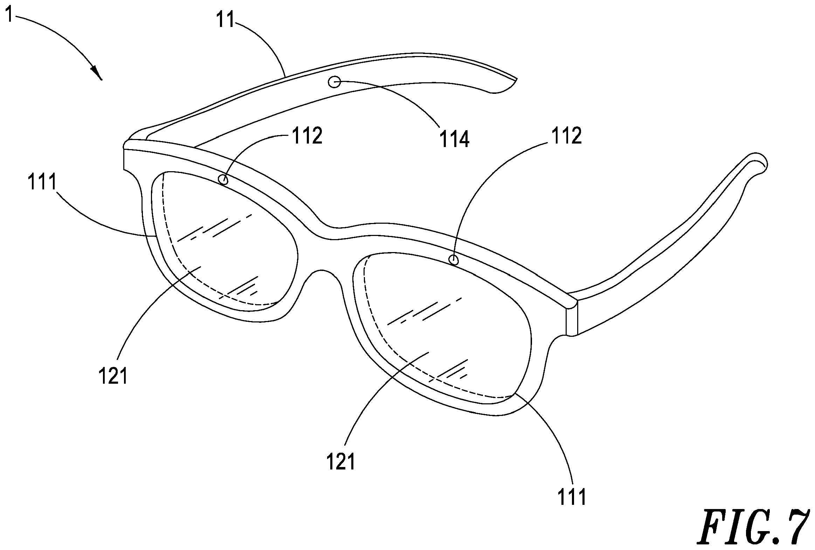

Moreover, as shown in Figure 7, the frame body 11 can be further installed with at least one or else more than one sensor device 114 electrically connected to the processor, in which the sensor device 114 can be a sensor capable of detecting temperature, heartbeat, blood pressure, perspiration or providing the step counting function, and the frame body 11 can be configured with sensor devices 114 having one or multiple identical or different features.

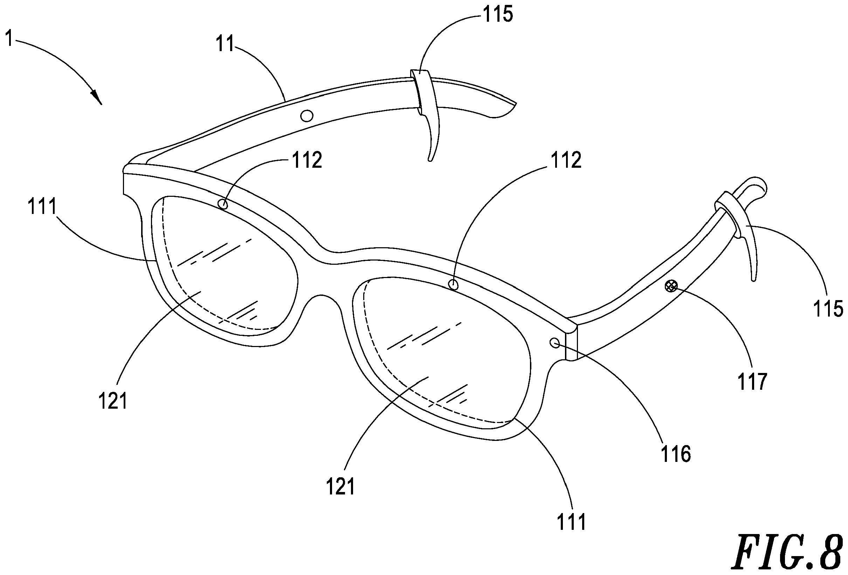

Besides, as shown in Figure 8, the frame body 11 is further configured with at least one or else more than one ear mountable device 115 electrically connected to the processor 113, in which the ear mountable device 115 can be directly connected to the power supply socket (not shown), and has a built-in battery (not shown) for providing electric power to the power supply module 1135 by way of the power supply socket.

Furthermore, as shown in Figure 8, the frame body 11 may be further installed with at least one microphone device 116 and speaker device 117 electrically connected to the processor 113.

In comparison with other conventional technologies, the eyeglasses structure enabling image enhancement according to the present invention provides the following advantages:
1. According to the present invention, a transparent display is jointly installed onto the lens of the common eyeglasses worn by general users, then the processor in the frame of the eyeglasses can perform image clarification processes on the image captured and forwardly extended from the frame in order to improve its resolution, and output the synchronously clarified image to the transparent display such that the image actually seen by the eyeball of the user through the lens can overlap with the synchronously clarified image shown on the transparent display thereby clarifying the scenery image observed by the eyeball of the user through the lens.
2. The present invention can render images on the retina to assist users having ocular diseases such that the eyesight can be improved without wearing eyeglasses equipped with curved lenses.
3. The image capture device according to the present invention further provides a zoom in / zoom out function, just like a camera, thereby enlarging an image to be captured at a remote position (similar to a telescope) or directly magnifying an image at a closer distance (similar to a magnifier) such that clear images can be successfully captured no matter at a farther or a closer distance.
4. The present invention allows a user to observe images that his/her own eyeballs can see such that the field of view may extend farther thereby clearly watching the view in front of the eyeball even for the scenery image exceeding the visible range of the eyeball.

Although the present invention has been disclosed through the detailed descriptions of the aforementioned embodiments, such illustrations are by no means used to restrict the present invention. Skilled ones in relevant fields of the present invention can certainly devise any applicable alternations and modifications after comprehending the aforementioned technical characteristics and embodiments of the present invention without departing from the spirit and scope thereof. Hence, the scope of the present invention to be protected under patent laws should be delineated in accordance with the claims set forth hereunder in the present specification.

## Claims

1. An eyeglasses structure enabling image enhancement, comprising:
a frame body, the interior of which being connected to a processor including:
a central processing module, used to control entire operations of the processor;
an image processing module, connected to the central processing module, in which the image processing module is used to perform image clarification processes on the externally captured image information in order to improve its resolution;
an image output module, connected to the central processing module and the image processing module in order to output the image-clarified externally captured image information as a synchronously clarified image;
a remote connection module, connected to the central processing module thereby performing remote connections by means of wireless connection technologies;
a power supply module, connected to the central processing module thereby connecting to an external device so as to store and supply electric power required for operations of the processor;
two lens bodies, combined with the frame body and having a first surface and a second surface, wherein the distance between the second surface and the eyeball of a user is smaller than the distance between the first surface and the eyeball of the user;
at least two transparent displays, respectively combined with the first surface, the second surface or the first and the second surfaces of the two lens bodies, and electrically connected to the image output module of the processor thereby displaying in real time the synchronously clarified image;
at least one or else more than one image capture device, combined onto the frame body and electrically connected to the image processing module of the processor in order to capture the image forwardly extended from the frame body and convert the image into the externally captured image information for transferring to the image processing module; and
the image actually seen by the eyeball of the user through the lens body can overlap with the synchronously clarified image displayed on the two transparent displays thereby clarifying the scenery image seen by the eyeball of the user through the lens body.

2. The eyeglasses structure enabling image enhancement according to Claim 1, wherein the processor further comprises a capture angle adjustment module which is electrically connected to the central processing module and the image capture device in order to adjust the angle of the captured image such that the image seen at the perspective of the eyeball can be of the same angle as the perspective of the image captured by the image capture device and forwardly extended from the frame body thereby that the image actually seen by the eyeball of the user through the lens body can overlap with the synchronously clarified image displayed on the two transparent displays.

3. The eyeglasses structure enabling image enhancement according to Claim 2, wherein the capture angle adjustment module can be pre-configured with a fixed eyeball perspective angle and perform pre-adjustments on the angle of the captured image based on the fixed eyeball perspective angle such that the image seen at the perspective of the eyeball can be of the same angle as the perspective of the image captured by the image capture device and forwardly extended from the frame body.

4. The eyeglasses structure enabling image enhancement according to Claim 3, wherein the pre-configured eyeball perspective angle is the direct view angle.

5. The eyeglasses structure enabling image enhancement according to Claim 2, 3 or 4, further capable of performing connections to the remote connection module of the processor by means of a software installed within an electronic device and transferring control commands for adjusting the angle of the captured image to the capture angle adjustment module by way of the central processing module in order to remotely adjust the angle of the captured image.

6. The eyeglasses structure enabling image enhancement according to Claim 1, wherein the processor further comprises an output image adjustment module which is electrically connected to the central processing module and the image output module thereby adjusting the display status of the synchronously clarified image shown on the transparent display.

7. The eyeglasses structure enabling image enhancement according to Claim 6, wherein the remote connection module of the processor can be connected to a cloud platform such that the cloud platform can transfer the digital display data to the processor and, through the output image adjustment module, the digital display data transferred by the cloud platform can be conjunctively shown over the synchronously clarified image on the transparent display.

8. The eyeglasses structure enabling image enhancement according to Claim 7, wherein the digital display data of different angles can be respectively shown on different transparent displays in order to render image effects presenting a depth of field or stereo view.

9. The eyeglasses structure enabling image enhancement according to Claim 7, wherein the processor can further transfer the externally captured image information to the cloud platform via the remote connection module.

10. The eyeglasses structure enabling image enhancement according to Claim 6, wherein the display status shown on the synchronously clarified image of the transparent display that the output image adjustment module can adjust may include adjusting multiple display perspectives, adjusting display location, adjusting display size, adjusting wide angle, adjusting display contrast or adjusting display brightness.

11. The eyeglasses structure enabling image enhancement according to Claim 6, wherein if any word or any replaceable object exists on the synchronously clarified image, then the output image adjustment module can replace the word in the synchronously clarified image shown on the transparent display with a clear word or replace the object with a built-in object.

12. The eyeglasses structure enabling image enhancement according to Claim 6, wherein if the synchronously clarified image is a dimmed image, then the output image adjustment module can perform light compensations to the synchronously clarified image shown on the transparent display.

13. The eyeglasses structure enabling image enhancement according to Claim 6, 7 ,8 or 9, further capable of performing connections to the remote connection module of the processor by means of a software installed within an electronic device and transferring control commands for adjusting the display status of the synchronously clarified image to the output image adjustment module by way of the central processing module in order to remotely adjust the display status of the output image.

14. The eyeglasses structure enabling image enhancement according to Claim 1, wherein the lens body is a flat lens.

15. The eyeglasses structure enabling image enhancement according to Claim 1, wherein the lens body is a curved lens.

16. The eyeglasses structure enabling image enhancement according to Claim 1, wherein the frame body is further configured with at least one or else more than one sensor device electrically connected to the processor.

17. The eyeglasses structure enabling image enhancement according to Claim 1, wherein any one of the sensor devices may be a sensor capable of detecting temperature, heartbeat, blood pressure, perspiration or providing a step counting function.

18. The eyeglasses structure enabling image enhancement according to Claim 1, wherein the frame body is further configured with at least one or else more than one ear mountable device electrically connected to the processor, and the ear mountable device has a built-in battery thereby providing electric power to the power supply module.

19. The eyeglasses structure enabling image enhancement according to Claim 1, wherein the frame body is further configured with at least one microphone device electrically connected to the processor, and the microphone device can transfer voice signals to the processor thereby voice controlling the operations of the processor.

20. The eyeglasses structure enabling image enhancement according to Claim 1, wherein the frame body is further configured with at least one speaker device electrically connected to the processor.

21. The eyeglasses structure enabling image enhancement according to Claim 1, wherein at least two image capture devices can be further used to respectively capture images of different angles, and the images of different angles can be combined by the processor into a stereo image message or an image message presenting a depth of field.

22. The eyeglasses structure enabling image enhancement according to Claim 1, wherein the export image adjustment module can also process the image in an approach of matrix or array such that the image exported to the transparent display exhibits the image focusing effect.

23. The eyeglasses structure enabling image enhancement according to Claim 1, wherein, in case of the multi-layered transparent display, suppose the output is fed to one of such layers in the transparent display, the images in any two layers or any two or more layers are processed in an approach of array or matrix thereby achieving the multiple image focusing effect.

24. The eyeglasses structure enabling image enhancement according to Claim 1, wherein the lens body or the transparent display can guide light by means of the collimation technology.

25. The eyeglasses structure enabling image enhancement according to Claim 24, wherein the collimation technology can achieve the objective of light guiding by means of microlens or light well.

26. The eyeglasses structure enabling image enhancement according to Claim 25, wherein the microlens can be further chamfered to adjust the direction of collimated light.

27. The eyeglasses structure enabling image enhancement according to Claim 24, wherein the lens body or the transparent display can be further chamfered to adjust the direction of collimated light such that two or more images can be overlapped.

28. The eyeglasses structure enabling image enhancement according to Claim 1, wherein, during the manufacture process of the transparent display, it is possible to employ the collimation technology or microlens technology such that the ex-factory transparent display enables the light guiding effect.
